# EUROPÄISCHE PATENTANMELDUNG

(11) **EP 1 510 198 A1**
(43) Veröffentlichungstag der Anmeldung: **02.03.2005**
(21) Anmeldenummer: 04016166.3
(22) Anmeldetag: 09.07.2004
(51) Int. Cl.: A61K 6/10

(54) **Mit Wasser aushärtende Alginatzusammensetzungen**

(30) Priorität: 29.08.2003 DE 10340334
(71) Anmelder: Cavex Holland BV, NL-2031 WH Haarlem (NL)
(72) Erfinder: Ippel, Dick, 2015 KL Haarlem (NL); Timmerman, Gerrit, 2013 BP Haarlem (NL); Timmermans, Johannes Wilhelmus, 6714GG Ede (NL); Van Soest, Jeroen Johannes Gerardus, 6703GL Wageningen (NL)
(74) Vertreter: Kühn, Hans-Christian

(57) **Zusammenfassung**

Mit Wasser aushärtende Alginatzusammensetzungen, enthaltend als Komponenten neben anderen
10 - 15% mindestens eines Akalimetallalginats,
5 - 15 % Calciumsulfathemihydrat oder -dihydrat oder Mischungen davon
0.1 - 2 % Natrium- oder Kaliumphosphat oder -pyrophosphat,
0 - 5% Magnesium- oder Zinkoxid,
0 - 4% Natrium- oder Kaliumsilicofluorid oder -fluorotitanat,
50 - 80 % einer Mischung von
   **A** Zellulose oder mindestens einem wasserunlöslichen Zellulosederivat, und
   **B** Stärke oder mindestens einem wasserunlöslichen Stärkederivat; sowie
0 - 30 % anorganischer Füllstoff aus der Gruppe: Diatomeenerde, Perlit, Talk oder Tonerden eignen sich besonders als Dental-Abformmassen.

## Beschreibung

Die Erfindung betrifft mit Wasser aushärtende Alginatzusammensetzungen.

In der Zahnheilkunde werden solche Zusammensetzungen als Abformmaterial verwendet, um Gebißabdrücke für die Modellherstellung zu nehmen. In der Kosmetik benutzt man sie für Gesichtsmasken. Es gibt schon seit langem Produkte, bei denen die Abbindereaktion zwischen Alginaten und Calcium oder ähnlichen polyvalenten Kationen genutzt wird. In der Regel werden diese Produkte in Pulverform angeboten und vom Anwender mit Wasser zu einer Paste vermischt. Im Munde des Patienten härten sie zu einer elastischen, formtreuen Masse.

Die reaktiven Komponenten der Abformmaterialien bestehen im wesentlichen aus einem löslichen Natrium- oder Kaliumalginat, einem langsam löslichen Calciumsalz als Geliermittel, einem Phosphat als Verzögerer sowie Puffersubstanzen zur Steuerung des pH, die zu einer schnelleren Aushärtungsreaktion führen und die Kompatibilität zum Gips, der für die Modellherstellung verwendet wird, erhöhen.

Die Verwendung natürlicher oder chemisch modifizierter wasserlöslicher Polysaccharide wie Pektin, Carboxymethylzellulose, Guarmehl und seiner Derivate, Johannisbrotkernmehl und Xanthangummi ist in Abformmaterialien bekannt. Diese Stoffe werden oft zusammen mit dem oder als Ersatz für das Alginat verwendet (siehe z.B. US 2,628,153 oder US 6,509,390).

Weitere Komponenten sind inerte Füllstoffe. Sie dienen zunächst der Bildung einer homogenen Paste und nach dem Abbinden zur Stabilisierung des Gels. Ohne solche Füllstoffe wäre das Gel nicht stark genug, die Mundsituation zuverlässig abzubilden. Im allgemeinen benutzt man mineralische anorganische Füllstoffe wie Diatomeenerde, Perlit, Talk oder Tonerden.

Ein Nachteil dieser Naturstoffe ist, dass ihre Eigenschaften - wie Farbe oder Wasseraufnahmevermögen - selbst bei Produkten derselben Herkunft in weiten Grenzen schwanken, was die Eigenschaften des Endprodukts erheblich beeinträchtigen kann. Hinzu kommt, dass die Naturstoffe nicht in unbegrenzten Mengen zur Verfügung stehen und eines Tages knapp werden. Ein weiterer Nachteil ist, dass einige der Füllstoffe als Stäube eingeatmet ein Gesundheitsrisiko darstellen können. Schließlich sind die inerten Füllstoffe nicht biologisch abbaubar und können bei der Entsorgung ein Problem darstellen.

Aufgabe der Erfindung ist es somit, Füllstoffe aufzufinden, welche diese Probleme zumindest teilweise beseitigen.

Erfindungsgemäß wird die Aufgabe durch die Verwendung wasserunlöslicher, nachwachsender Materialien als zumindest teilweiser Ersatz für anorganische Füllstoffe in Alginatabformmaterialien gelöst.

Die Erfindung betrifft demzufolge mit Wasser aushärtende Alginatzusammensetzungen enthaltend
10 - 15% mindestens eines Akalimetallalginats,
5 -15 % Calciumsulfathemihydrat oder -dihydrat oder Mischungen davon
0.1 - 2 % Natrium- oder Kaliumphosphat oder -pyrophosphat,
0 - 5 % Magnesium- oder Zinkoxid,
0 - 4 % Natrium- oder Kaliumsilicofluorid oder -fluorotitanat,
50 - 80 % einer Mischung von
**A** Zellulose oder mindestens einem wasserunlöslichen Zellulosederivat, und
**B** Stärke oder mindestens einem wasserunlöslichen Stärkederivat, sowie
0 - 30 % anorganischer Füllstoff aus der Gruppe: Diatomeenerde, Perlit, Talk oder Tonerden.

Wie oben erwähnt, bestehen Alginatabformmaterialien im wesentlichen aus den Komponenten Gelbildner (Alginat), Geliermittel (Calciumsulfat), Verzögerer (Natriumphosphat), den pH steuernde Puffer (Magnesium- und/oder Zinkoxid und/oder -carbonat, Kalium oder Natriumsilicofluorid oder -fluorotitanat), und Füllstoff(e).

Die Art und Eigenschaften der Füllstoffe sind für viele Eigenschaften des Abfommaterials bestimmend. Zunächst muss sich eine Paste bilden, die sich an den Abformlöffel anpasst, nicht aber aus diesem in den Rachen des Patienten herausfließt. Die Anmischzeit für das Pulver-Wasser-Gemisch ist kurz. Es sollte sich binnen einer Minute ein glatte homogene Paste bilden. Das Abformmaterial sollte ferner feinste Details abbilden können. Dazu ist die ISO Norm 1563 einzuhalten, nach der eine 50 µm breite Schramme in einem Metallblock vollständig reproduziert werden muss. Die Abformung muss stark und elastisch genug sein, damit sie ohne Abreißen oder Verformungen aus dem Mund entfernt werden kann. Die Abformung muss gegen Schrumpfen oder Ausbluten der Flüssigkeit (Synerese) stabil sein.

Füllstoffe, die diesen Anforderungen genügen sollen, sollten folgenden Eigenschaften aufweisen:
- Hohe Wasserabsorption, hohe Hydrophilie.
- Verstärkende Wirkung ohne Nebenreaktionen mit weiteren Komponenten.
- Farblosigkeit.
- Durchschnittliche Teilchengröße relativ weit unter 50 µm.
- Ungiftigkeit für Patienten und Behandler.

Es wurde nun gefunden, dass natürliche Polymere wie Zellulose und Stärke diese gewünschten Eigenschaften besitzen, ohne die weiter oben beschriebenen Nachteile aufzuweisen. Geeignet sind auch chemisch modifizierte Abkömmlinge dieser natürlichen Polymere. Pflanzliche Zellulose, besonders aus dem Holz von Bäumen, besteht aus langen Glucose-Ketten. Sie ist wasserunlöslich und semikristallin. Kommerziell sind Fasern (z.B. Arbocell, Rettenmaier) oder Pulver erhältlich. Bei dem Pulver (z.B. Avicel, FMC) handelt es sich um teilweise depolymerisiertes Material. Stärke ist in ihrer natürlich gewonnenen Form ebenfalls wasserunlöslich und besteht aus einer Mischung von Amylopektin und Amylose (z.B. Kartoffelstärke, Avebe). Natürlich gewonnene Stärke ist granular. Es gibt wie bei der Zellulose verschiedene Sorten mit verschiedener Zusammensetzung und Korngröße, je nach der pflanzlichen Herkunft aus z.B. Weizen, Mais, Kartoffeln, Reis oder Tapioka.

Von Zellulose oder Stärke sind eine Reihe chemischer Modifikationen bekannt, z.B. oxidierte Formen, Ester oder Ether. Gewöhnlich wird dadurch die Wasserlöslichkeit erhöht, und diese Materialien sind dann als Füllstoffe für Alginatabformmassen nicht mehr geeignet. Beispielsweise würde das Gel instabil oder die Oberfläche der Gipsmodelle würde durch lösliche Polysaccharide beeinträchtigt.

Stärke wurde bereits als Füllstoff in Abformmassen vorgeschlagen, die frei von Diatomeenerde sind (JP 02-304011).

Es wurde jedoch gefunden, dass Zusammensetzungen, die zusätzlich zur Stärke noch faserige Zellulose enthalten, überraschend überlegene Eigenschaften haben.

Ähnlich wie die mineralischen Füllstoffe bestimmen auch Stärke und Zellulose das Mischverhalten und die Geleigenschaften der entsprechenden Abformmassen. So sind Stärke und einige feinkristalline Zellulosearten sehr feine Pulver, die bei der Anmischung zum Klumpen neigen, was zu Löchern oder Buckeln beim Abdruck führt. Auf der anderen Seite führt die Verwendung von Faserzellulose allein zu keiner vollständig glatten Masse, und der Abdruck wird nicht präzise genug im Detail.

Bei den Zusammensetzungen der Erfindung kommt es somit wesentlich auf die ausgewogene Mischung der beiden organischen Füllerkomponenten, Zellulose und Stärke an.

Die Zusammensetzungen können noch Anteile der üblichen anorganischen Füllstoffe aus der Gruppe: Diatomeenerde, Perlit, Talk oder Tonerden enthalten, vorzugsweise in Mengen zwischen 5 und 30 %. Besonders bevorzugt sind diese anorganischen Füllstoffe jedoch ganz abwesend.

Besonders geeignete erfindungsgemäße Zusammensetzungen enthalten folgende Bestandteile:

| | |
|---|---|
| Natrium-/ Kaliumalginat | 10 -15 % |
| Calciumsulfat | 5 -15 % |
| Natrium-/ Kaliumphosphat | 0.1 -2,0 % |
| Kaliumfluortitanat / Natriumsilicofluorid | 1 - 4 % |
| Magnesium- / Zinkoxide | 1- 5 % |
| Stärke / mikrokristalline Zellulose | 0 - 70% |
| Zellulose faserig | 0 - 70 % |
| Staubverhinderndes Mittel (PEG. PPG, Öl) | 0 - 6 % |
| Farbstoffe | Spuren |
| Geschmackstoffe | Spuren |

Die Verwendung solcher Mischungen führt zu glatten und homogenen Abformpasten mit akzeptablen Anmischzeiten.

**Tabelle**

| Komponenten in % (m/m) | Beispiel 1 (Vergleich) | Beispiel 2 (Erfindung) |
|---|---|---|
| Natrium-/ Kaliumalginat | 13 | 13 |
| Calciumsulfat dihydrat | 10 | 10 |
| Natriumphosphat | 1 | 1 |
| Kaliumfluorotitanat | 2 | 2 |
| Magnesiumoxid | 2 | 2 |
| Diatomeenerde | 70 | |
| Stärke (Kartoffelstärke, Avebe) | | 43 |
| Zellulose faserig (Arbocell, Rettenmaier) | | 22 |
| Anti-Staub-Mittel | 2 | 2 |
| Farbstoff | Spuren | Spuren |
| Geschmackstoff | Spuren | Spuren |

23 g des Pulvers, sowohl nach Beispiel 1 als 2 der Tabelle, werden mit Wasser in Berührung gebracht und dann vermischt. Das Pulver benetzt leicht und binnen 40 Sekunden bildet sich eine glatte Paste. Die physikalischen Eigenschaften erfüllen ISO 1563. Damit zeigt sich die Eignung der erfindungsgemäßen Mischung des Beispiels 2 als Abformmaterial.

## Patentansprüche

1. Mit Wasser aushärtende Alginatzusammensetzungen enthaltend
10 - 15% mindestens eines Akalimetallalginats,
5 - 15 % Calciumsulfathemihydrat oder -dihydrat oder Mischungen davon
0.1 - 2 % Natrium- oder Kaliumphosphat oder -pyrophosphat,
0 - 5 % Magnesium- oder Zinkoxid,
0 - 4 % Natrium- oder Kaliumsilicofluorid oder -fluorotitanat,
50 - 80 % einer Mischung von
**A** Zellulose oder mindestens einem wasserunlöslichen Zellulosederivat, und
**B** Stärke oder mindestens einem wasserunlöslichen Stärkederivat; sowie
0 - 30 % anorganischer Füllstoff aus der Gruppe: Diatomeenerde, Perlit, Talk oder Tonerden.

2. Mit Wasser aushärtende Alginatzusammensetzungen enthaltend
10 - 15% mindestens eines Akalimetallalginats,
5 - 15 % Calciumsulfathemihydrat oder -dihydrat oder Mischungen davon
0.1 - 2 % Natrium- oder Kaliumphosphat oder -pyrophosphat,
0 - 5 % Magnesium- oder Zinkoxid,
0 - 4 % Natrium- oder Kaliumsilicofluorid oder -fluorotitanat,
50 - 80 % einer Mischung von
**A** Zellulose oder mindestens einem wasserunlöslichen Zellulosederivat, und
**B** Stärke oder mindestens einem wasserunlöslichen Stärkederivat, sowie
5 - 30 % anorganischer Füllstoff aus der Gruppe: Diatomeenerde, Perlit, Talk oder Tonerden.

3. Zusammensetzungen nach Anspruch 1 oder 2, die ab 10 Gew. % **B** und bis zu 60 Gew. % **A** enthält.

4. Zusammensetzungen nach einem der Ansprüche 1 bis 3, worin die Zellulose oder deren Derivate in Faser- oder Pulverform vorliegen.

5. Zusammensetzungen nach einem der Ansprüche 1 bis 4, worin die Stärke oder deren Derivate granular sind und aus Weizen, Kartoffeln, Reis, Mais oder Tapioka stammen.

6. Dentales Abformmaterial, im wesentlichen bestehend aus einer Zusammensetzung gemäß einem der Ansprüche 1 bis 5.

7. Dentales Abformmaterial gemäß Anspruch 6, enthaltend zusätzlich Farb- und Geschmacksstoffe.
